# EUROPEAN PATENT APPLICATION

(11) **EP 4 821 712 A1**
(43) Date of publication of application: **14.10.2026**
(21) Application number: 25169549.0
(22) Date of filing: 09.04.2025
(51) Int. Cl.: B60H 1/00, A61L 9/20, B60H 3/00, F24F 8/22

(54) **AIR CONDITIONING SYSTEM FOR VEHICLES**

(71) Applicant: Teleco Sat Equipement Srl, 48022 Lugo (RA) (IT)
(72) Inventor: MAGRI, Marco, 48018 Faenza (RA) (IT)
(74) Representative: Modiano, Micaela Nadia

(57) **Abstract**

A service air conditioning system for vehicles provided with a living unit (101), of the type operable with the engine switched off and which comprises at least four service components, constituted by a compressor (11), a condenser (12), a throttle element (13), and an evaporator (14), arranged along a closed circuit for the execution of a refrigeration cycle. The four service components are divided between a first functional unit (16), comprising at least the evaporator (14), and a second functional unit (17), distinct from the first functional unit (16) and comprising at least the compressor (11) and the condenser (12); the first functional unit (16) is accommodatable in the living unit (101) of the vehicle, while the second is accommodatable in an engine compartment (102) of the vehicle;
the service system (10) comprises a sterilizer (18) configured to sterilize the air treated by the evaporator (14).

## Description

The present invention relates to an air conditioning system for vehicles, a related vehicle, and a method for installing an air conditioning system for vehicles.

As is known, all or almost all motor vehicles in circulation have an air conditioning (or climate control) system as standard, which is powered by the same engine that propels the vehicle and the function of which is to regulate the temperature in the passenger compartment, ensuring the comfort of the driver and of the other occupants during the trip.

The system can operate only when the vehicle is underway, precisely because it is powered by the internal combustion engine of the vehicle, which, in particular, rotates the compressor responsible for the forced circulation of the refrigerant fluid inside a closed circuit, so as to execute a refrigeration cycle to subtract heat from the passenger compartment and convey it outside.

In the vast and extremely varied category of motor vehicles, an undoubtedly prominent role is occupied by what are known as "recreational vehicles", the definition of which includes for example camper vans, caravans, motor homes, and more generally all those vehicles designed to enable a stay for its occupants, inside a living unit that can be variously fitted out (and optionally separate from the driver's cab).

In these motor vehicles, the need is often felt to control the temperature and in general the environmental conditions of the living unit even while the engine is off, but this functionality cannot be performed by the system described above, which as has been seen operates only when the engine is running.

The use is therefore known of a second, service system, which in turn is constituted by an air conditioner that is independent of the one described previously, and which can be operated even, and especially, when the engine is off.

Such solutions are not however devoid of drawbacks, and in particular manufacturers are constantly seeking solutions that improve the use experience of climate control systems by the people staying in the living unit.

The aim of the present invention is to solve the above mentioned problems, by providing an air conditioning system for vehicles provided with a living unit, operable with the engine switched off, which offers an optimal use experience to people staying in the living unit.

Within this aim, an object of the invention is to provide a vehicle provided with a living unit, equipped with an air conditioning system operable with the engine switched off, that offers an optimal use experience to people staying in the living unit.

Another object of the invention is to provide a method for installing an air conditioning system, on vehicles provided with a living unit, that makes it possible to offer an optimal use experience to people staying in the living unit.

Another object of the invention is to provide a system, a vehicle, and a method that overcome the drawbacks of the prior art in a different manner to any existing solutions.

Another object of the invention is to provide a system, a vehicle, and a method that are highly reliable, safe in use and can be provided or implemented relatively simply and at low cost.

This aim and these and other objects which will become better apparent hereinafter are achieved by an apparatus according to claim 1, by a vehicle according to claim 7 and by a method according to claim 12, optionally provided with one or more of the characteristics of the dependent claims.

Further characteristics and advantages of the invention will become more apparent from the description of some preferred, but not exclusive, embodiments of the system, of the vehicle and of the method according to the invention, which are illustrated by way of non-limiting example in the accompanying drawings wherein:
Figure 1 schematically illustrates a first configuration of the system according to the invention, which can be installed by carrying out the method according to the invention;
Figure 2 schematically illustrates a second configuration of the system according to the invention, which can be installed by carrying out the method according to the invention;
Figure 3 schematically illustrates a third configuration of the system according to the invention, which can be installed by carrying out the method according to the invention;
Figure 4 schematically illustrates a fourth configuration of the system according to the invention, which can be installed by carrying out the method according to the invention;

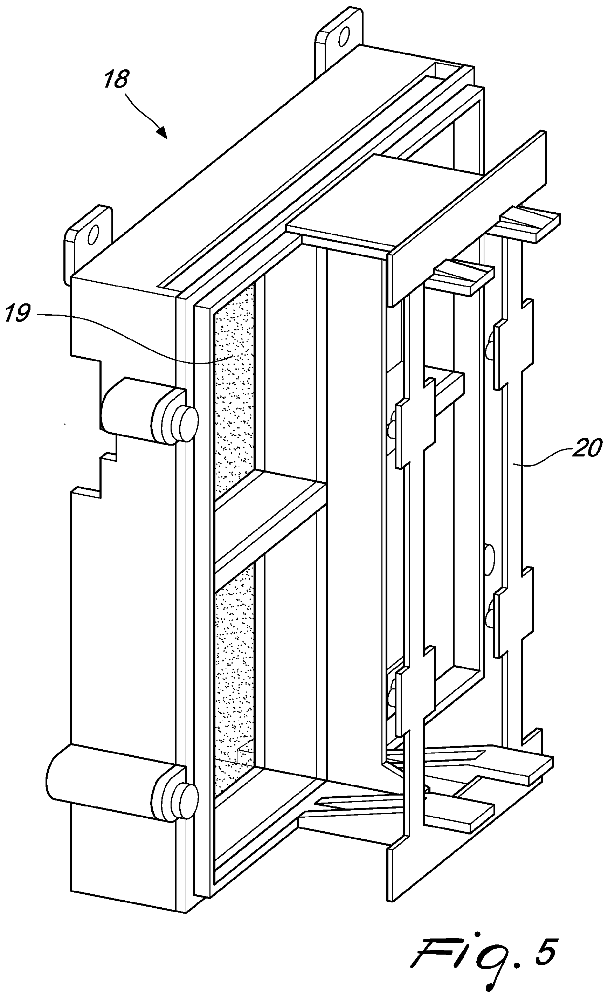

Figure 5 is a perspective view of the sterilizer of the system according to the invention;

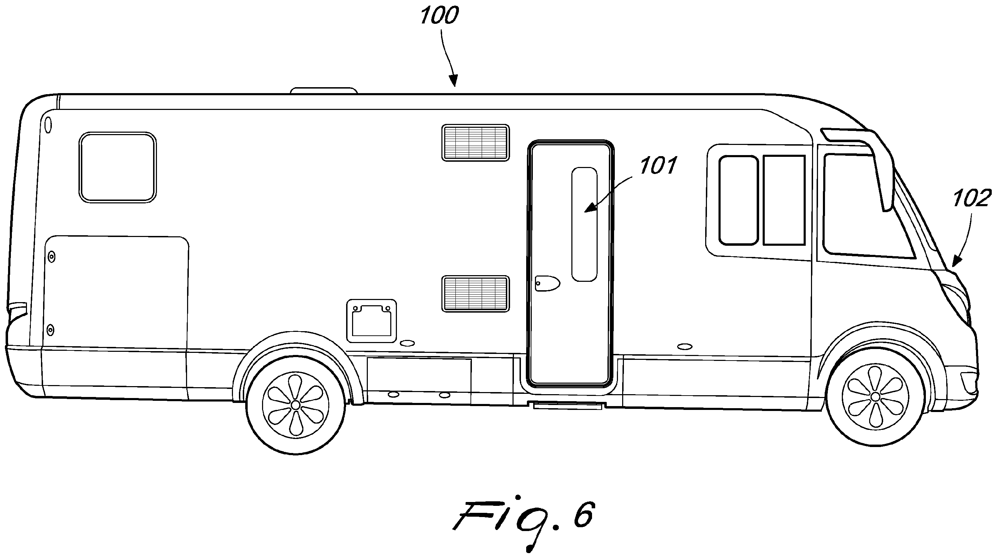

Figure 6 is a perspective view of a vehicle according to the invention.

With reference to the figures, the reference numerals 10 and 100 designate respectively a service air conditioning system (operable with the engine switched off) for vehicles provided with a living unit 101 and the vehicle itself, which constitute the subject matter of the present discussion and of the protection that is claimed herein, which is also claimed for a method for installing the service system 10 (operable with the engine switched off) in the vehicle 100.

It should be noted that in the present description the expression "air conditioning system" should be understood as entirely equivalent to "climate control system", or "air conditioner", or "conditioner", and the specifics, peculiarities and features described below, as well as the protection claimed, can extend indifferently to any of the aforesaid terms.

The vehicles 100 can be camper vans, livable vans, caravans, motor homes, and all other vehicles 100 that incorporate a living unit 101 (overall these vehicles are identified in English by the category of "recreational vehicles"), where such a living unit 101 is a compartment fitted out (permanently) to enable a stay for its occupants.

The scope of protection should be understood to also include vehicles 100 other than "recreational vehicles" properly speaking, as long as they are provided with a living unit 101 (as is the case with some semi-trailer tractors, or trucks, for example).

The vehicle 100 comprises first of all, and entirely conventionally, a primary air conditioning system 20, designed to operate with the engine running (when the vehicle 100 is in motion or underway), in that it is powered by the same engine (internal combustion or other type) responsible for the propulsion of the vehicle 100 and accommodated in an engine compartment 102.

Furthermore, the vehicle 100 comprises the service system 10, powered independently of the primary system 20 so that it can be actuated and operated with the engine switched off, when the occupants are staying in the vehicle 100 and in particular in the living unit 101.

The service system 10 comprises at least four service components, constituted by a compressor 11, a condenser 12, a throttle element 13, and an evaporator 14, which are arranged along a closed circuit (sealed) for the execution of a refrigeration cycle (by means of a gas or other refrigerant fluid, circulating in such circuit and in the pipes that go to make it up and which connect the aforementioned components to each other).

The refrigeration cycle thus makes it possible to cool the environment exposed to the evaporator 14, but it is also possible to use the service system 10 as a heat pump.

The evaporator 14 of the service system 10 can comprise the electrical and/or electronic components for controlling the service system 10, and also the user command interface and the vents for the distribution of the conditioned/climate-controlled air.

Likewise, the primary system 20 with which the vehicle 100 is equipped comprises in turn at least four primary components, constituted by a compressor 21, a condenser 22, a throttle element 23, and an evaporator 24, which are also arranged along a closed circuit (sealed and at least partially separate from the previously-mentioned circuit, as will be made clearer in the following pages), for the execution of a refrigeration cycle (by means of a gas or other refrigerant fluid, circulating in such circuit and in the pipes that go to make it up and which connect the aforementioned components to each other).

The compressors 11, 21 basically operate like a pump, moving the gas to the other components; in order to obtain the desired climate control, each compressor 11, 21 must be powered by the engine of the vehicle 100 (in the case of the primary system 20) or by other means (in the case of the service system 10). In the condensers 12, 22 (optionally associated with fans 12a, 22a) the phase transition occurs, at high pressure, from gas to liquid (with release of heat, externally); in the throttle elements 13, 23 the pressure is lowered and, finally, in the evaporators 14, 24 a new phase transition occurs, at low pressure, from liquid to gas, with absorption of heat. Downstream of the evaporators 14, 24, the coolant is therefore again in the gaseous phase (at low pressure) and can be sent back into the respective compressors 11, 21 to begin a new cycle.

The throttle element 13, 23 can be a capillary, an expansion valve or a choke valve (with the capacity to control throttle), or the like.

There can also be a filtration device 15, 25 outside a respective compressor 11, 21 (arranged proximate to the latter or at a distance from it, along the respective circuit), the function of which is to prevent the coolant liquid in liquid form from reentering the compressor 11, 21.

In any case, up to this point we have discussed operating modes that are well known, and there is no need to dwell on them further: any other implementation detail (apart from the peculiarities illustrated below) can be chosen to be one of the conventional solutions that the person skilled in the art would in each instance deem best suited for the purpose.

Furthermore, the four service components are divided between a first functional unit 16, which comprises at least the evaporator 14 (of the service system 10), and a second functional unit 17, which is (physically) distinct from the first and comprises at least the compressor 11 and the condenser 12. The element 13 can be comprised in the first functional unit 16, as in the accompanying figures, but the possibility is not ruled out of its forming part of the second, while in any case remaining within the scope of protection claimed herein.

So, effectively, the service system 10 is configured like a domestic "split" air conditioner/climate control system, in which, as is known, a first unit contains (at least) the compressor and a second unit (the "split"), physically separate and distinct from the first unit, contains (at least) the evaporator.

Thus, the first functional unit 16 can be accommodated in the living unit 101 of the vehicle 100, while the second functional unit 17 can be accommodated in the engine compartment 102 of the vehicle 100.

The pipes of the circuit of the service system 10 are distributed appropriately, so as to connect the various components to each other.

In the present discussion, the term "functional unit" should be understood to mean a set of components enclosed in a specific environment (and optionally contained in a dedicated shell), chosen from either the living unit 101 or the engine compartment 102.

According to the invention, the service system 10 comprises a sterilizer 18, which is configured to sterilize the air treated (or processed) by the evaporator 14.

The sterilizer 18 adds an additional and peculiar functionality to the service system 10, in that it makes it possible to sanitize the air that circulates in the living unit 101 (the air treated by the evaporator 14), so ensuring an undoubted benefit for the occupants. The set aim is therefore achieved from this point onward, in that the service system 10 (and also the vehicle 100 in which it is installed) offers an optimal use experience to people staying in the living unit 101.

In particular, in the preferred, but non-limiting, embodiment of the application of the invention, the sterilizer 18 comprises a catalytic filter 19 and an LED light source 20, which is configured to activate a reaction on the catalytic filter 19, which is adapted to sterilize the air (and sanitize it).

More specifically, the LED light source 20 is of the ultraviolet radiation (UV) type.

However, the possibility is not ruled out of using sterilizers 18 that comprise different types of LED light sources 20 (or other types of light source, or even providing the sterilization in another manner, without using light), while remaining within the scope of protection claimed herein.

Advantageously, the sterilizer 18 can be mounted at the air entry point in a fan 14a of the evaporator 14, so as to ensure the sanitization of the entire mass of air treated by the evaporator 14.

Usefully, the light source 20 can be configured to be actuated simultaneously with the fan 14a.

In this way, the activation of the reaction on the catalytic filter 19, by the light source 20, occurs every time the ventilation of the living unit 101 (ensured by the fan 14a) is active, so as to also have the sanitization of the air active simultaneously.

Positively, the service system 10 can comprise a current inverter which is functionally associated with the compressor 11 (of the service system 10) and which can be connected to an alternator of the vehicle 100, so as to be able to power the compressor 11 when the vehicle 100 is underway. In particular, by virtue of the inverter it is possible to convert the 12V direct voltage output by the alternator of the engine to a 230V alternating voltage at 50Hz.

It is in any case envisaged that the compressor 11 of the service system 10 can be powered with mains voltage (i.e. for example 230V at 50Hz). The possibility is not ruled out of using another, different power source (in any case other than the engine of the vehicle 100), preferably but not necessarily of the electric type.

The service system 10 can be controlled from a user interface provided on a panel (locally) arranged in the vehicle 100. Alternatively, or as an option complementing that just described, the service system 10 can be controlled by means of a contactless information transmission technology.

In more detail, for example the possibility is envisaged for the service system 10 to be controlled by a user with an infrared remote control or over Wi-Fi, Bluetooth^{®}, or CI-BUS (the standard communication protocol for recreational vehicles, based on the LIN protocol).

As mentioned above, in addition to the service system 10, the subject matter of the protection claimed herein also comprises a vehicle 100 of the type mentioned above.

The vehicle 100 is therefore provided with a living unit 101 and with an engine compartment 102; the vehicle 100 further comprises, first of all, a primary air conditioning system 20, operable only with the engine running and comprising at least four primary components constituted by a compressor 21, a condenser 22, a throttle element 23, and an evaporator 24.

According to the invention, the vehicle 100 also comprises a service air conditioning system 10 according to the description in the foregoing pages, which is operable with the engine switched off.

Effectively, therefore, protection is claimed herein for a service system 10 which is supplied separately and is intended to be installed on a vehicle 100, and also for a vehicle 100 equipped with such service system 10, whether it is sold as such by the manufacturer or fitted out with a service system 10 at another time.

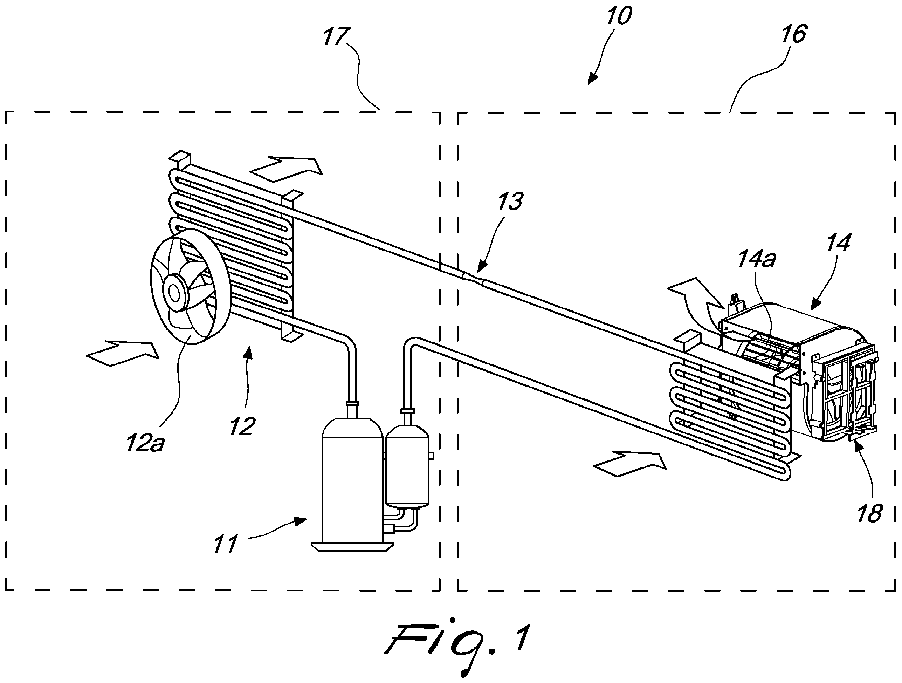

In a first possible configuration (of the systems 10, 20 and therefore of the vehicle 100), the four service components (of the service system 10, shown in Figure 1) are distinct from the respective four primary components (of the primary system 20). So in this case, the vehicle 100 is provided with two systems 10, 20 which are fully independent of each other and completely separate from each other.

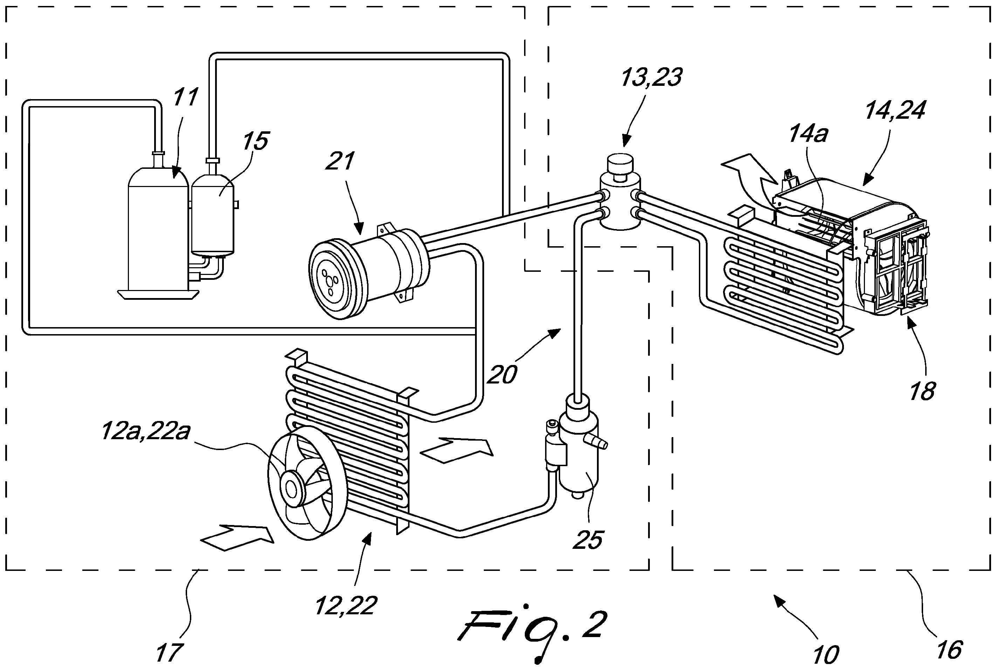

In a second possible configuration (of the systems 10, 20 and therefore of the vehicle 100), shown in Figure 2, the compressor 11 of the service system 10 is arranged in parallel with the compressor 21 of the primary system 20, so as to be able to use the condenser 22, the throttle element 23, and the evaporator 24 of the primary system 20 also, respectively, as the condenser 12, the throttle element 13, and the evaporator 14 of the service system 10. In this case, the sterilizer 18 therefore acts on the common evaporator 14, 24. In this second configuration, it is possible to use much of the primary system 20 of the vehicle 100 also for the service system 10 (and therefore for operation when the engine is switched off), in that, effectively, it is necessary to add (to a vehicle 100 provided with a primary system 20) only the compressor 11 of the service system 10, powered not by the engine of the vehicle 100 but by a different power source.

In the second configuration, just described, the existence of the service system 10 remains completely transparent to the user, in that the cool and sanitized air is introduced into the vehicle 100 preferably through the same ventilation vents that are also used to cool the environment when the vehicle 100 is running (the evaporator 14, 24 used is in fact, as mentioned, the same).

However, the possibility is not ruled out of also connecting the throttle element 13 of the service system 10 in parallel, so as to also keep the latter separate from the throttle element 23 of the primary system 20.

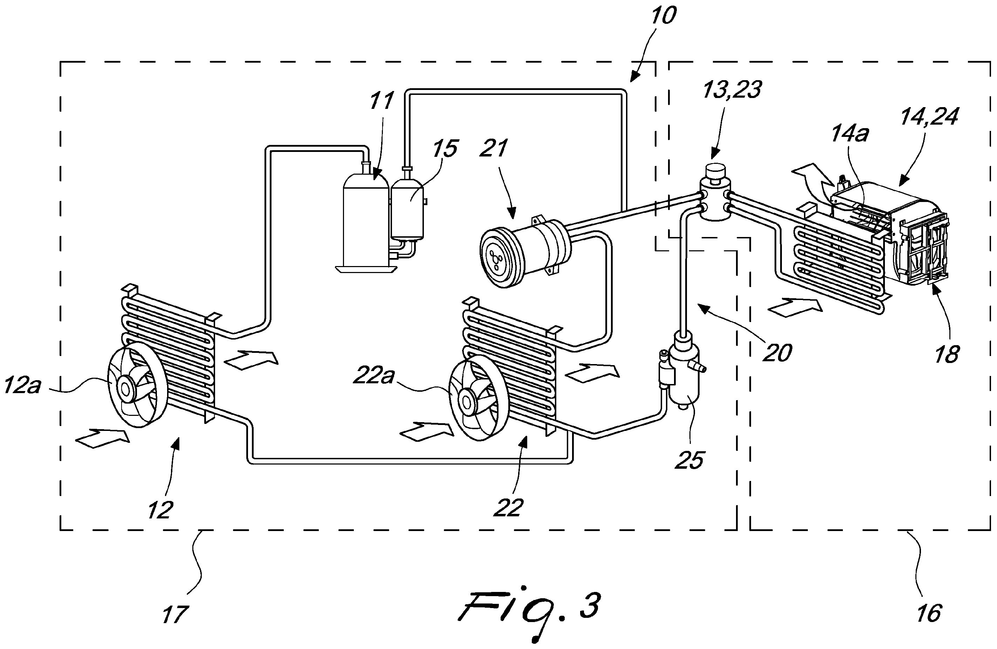

In a third possible configuration (of the systems 10, 20 and therefore of the vehicle 100), shown in Figure 3, the compressor 11 and the condenser 12 of the service system 10 are arranged in parallel with the compressor 21 and the condenser 22 of the primary system 20, so as to be able to use the throttle element 23 and the evaporator 24 of the primary system 20 also, respectively, as the throttle element 13 and the evaporator 14 of the service system 10. In this case too therefore, the sterilizer 18 acts on the common evaporator 14, 24. Such third configuration again makes it possible to use a significant part of the primary system 20 of the vehicle 100 also for the service system 10 (and therefore operable when the engine is switched off), in that, effectively, it is necessary to add the compressor 11 and the condenser 12 (which form a subgroup referred to in the sector as a "motor condensing unit") of the service system 10 (to a vehicle 100 fitted with a primary system 20), with the compressor 11 powered not by the engine of the vehicle 100 but by a different power source.

In this case too furthermore, as in the second configuration described above, effectively the existence of the service system 10 remains completely transparent to the user (in this configuration too, in fact, the evaporator 14, 24 used is the same).

However, the possibility is not ruled out of also connecting the throttle element 13 of the service system 10 in parallel, so as to also keep the latter separate from the throttle element 23 of the primary system 20.

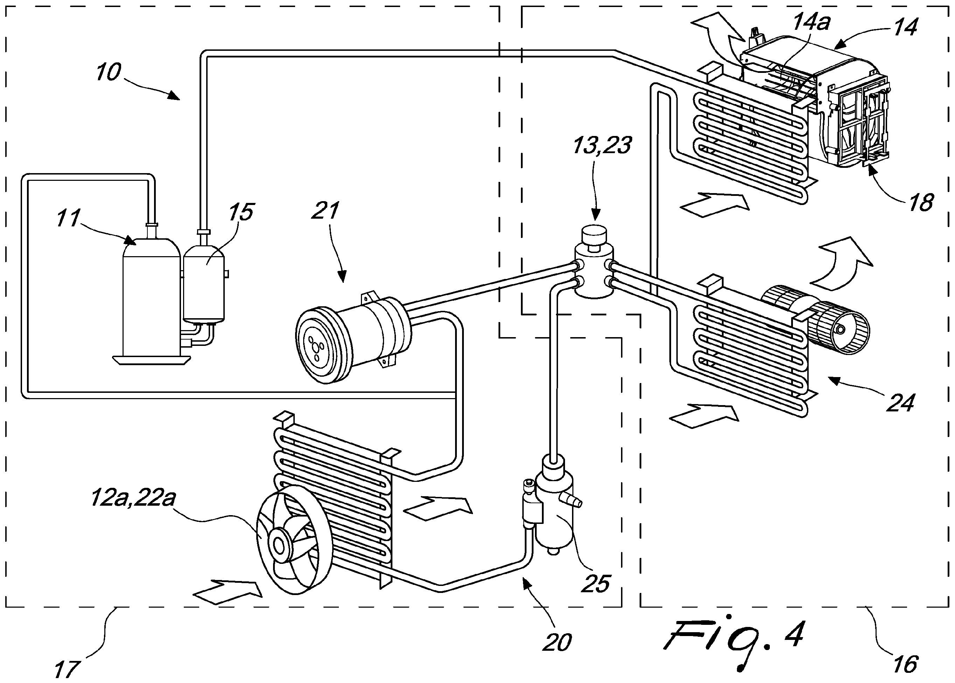

In a fourth possible configuration (of the systems 10, 20 and therefore of the vehicle 100), shown in Figure 4, the compressor 11 and the evaporator 14 of the service system 10 are arranged in parallel with the compressor 21 and the evaporator 24 of the primary system 20, so as to be able to use the condenser 22 and the throttle element 23 of the primary system 20 also, respectively, as the condenser 12 and the throttle element 13 of the service system 10.

Again, the possibility is not ruled out of also connecting the throttle element 13 of the service system 10 in parallel, so as to also keep the latter separate from the throttle element 23 of the primary system 20.

Such fourth configuration still makes it possible to use a significant part of the primary system 20 of the vehicle 100 also for the service system 10 (and therefore operable when the engine is switched off), in that it is necessary to add the compressor 11 and the evaporator 14 (equipped with a sterilizer 18) for the service system 10 (to a vehicle 100 fitted with a primary system 20), with the compressor 11 powered not by the engine of the vehicle 100 but by a different power source.

In this case, the user can have additional aeration vents in the vehicle 100, for the service system 10 only, but the possibility is not ruled out of using them for the primary system 20 as well.

In addition to the service system 10 and the vehicle 100, protection is also claimed herein for a method for installing the service air conditioning system 10, operable with the engine switched off, in a vehicle 100 provided with a living unit 101 and with a primary air conditioning system 20, operable only with the engine running.

The method according to the invention relates to the installation of a service system 10 according to what is described up to this point, on a vehicle 100 according to what is described up to this point.

In particular, therefore, each system 10, 20 comprises at least four components, constituted by a compressor 11, 21, a condenser 12, 22, a throttle element 13, 23, and an evaporator 14, 24, arranged along respective closed circuits for the execution of a refrigeration cycle.

The method entails arranging a first functional unit 16 of the service system 10, which comprises at least the evaporator 14 and preferably also the throttle element 13 (of the service system 10), in the living unit 101 of the vehicle 100, and a second functional unit 17 of the service system 10, separate from the first functional unit 16 and comprising at least the compressor 11 and the condenser 12 (of the service system 10), in an engine compartment 102 of the vehicle 100.

According to the invention, the method further entails equipping the service system 10 with a sterilizer 18 configured to actuate the sterilization of the air treated by the respective evaporator 14. The sterilizer 18 can also be provided in the ways explained above.

The use of the invention has been explained in the foregoing pages: the primary system 20 and the service system 10 execute, in a manner that is conventional per se, a refrigeration cycle that makes it possible to cool the environments inside the vehicle 100; in particular, the primary system 20 operates when the engine is running and therefore it performs its function when the vehicle 100 is underway, while the service system 10 comprises a compressor 11 which is not powered by the engine and which can therefore ensure the climate control of the living unit 101 when the engine is switched off, during the stay of the occupants.

In this context, in an entirely peculiar manner the choice to use a sterilizer 18 makes it possible to sanitize the air treated by the evaporator 14, thus ensuring an optimal use experience for the individuals inside the vehicle 100.

The choice to distribute the components of the service system 10 into two distinct and physically separate functional units 16, 17 ensures further benefits.

A good deal of the service system 10 is in fact located in the engine compartment 102, in an area that in any case cannot be used for the stay (evidently) and is certainly external to the living unit 101, and therefore the compressor 11 and the condenser 12 of the service system 10 do not reduce the space available to the occupants of the living unit 101. Furthermore, the fact that the compressor 11 of the service system 10 (the noisiest component) is located some distance away and in a position protected from the living unit 101 is an additional assurance of comfort for the users. In particular, the condenser 12 of the service system 10 can be fixed inside the engine compartment 102 using any preexisting holes present, and therefore without altering the mechanics of the chassis of the vehicle 100.

The evaporator 14 (the dimensions of which are contained with respect to the entire service system 10) can be in turn arranged in an area that is not accessible to the user and in any case the bulk is limited, with respect to conventional solutions that resort to single-unit air conditioners accommodated completely in what is intended to be living space.

Evidently, the service system 10 is particularly versatile, in that it can be installed on any vehicle 100, without obstructing windows and even in the presence of reduced-size roofs or lifting roofs, the functionality of which is therefore not affected in any way.

The possibility to use some of the components already used for the primary system 20 to provide the service system 10 (as seen in some of the configurations illustrated in the foregoing pages) ensures the low cost of the solution and greater ease and rapidity of installation.

The invention thus conceived is susceptible of numerous modifications and variations, all of which are within the scope of the appended claims. Moreover, all the details may be substituted by other, technically equivalent elements.

In practice the materials employed, provided they are compatible with the specific use, and the contingent dimensions and shapes, may be any according to requirements and to the state of the art.

Where technical features mentioned in any claim are followed by reference signs, such reference signs have been inserted for the sole purpose of increasing the intelligibility of the claims and accordingly such reference signs do not have any limiting effect on the interpretation of each element identified by way of example by such reference signs.

## Claims

1. A service air conditioning system for vehicles (100) provided with a living unit (101), of the type operable with the engine switched off and which comprises at least four service components, constituted by a compressor (11), a condenser (12), a throttle element (13) and an evaporator (14), arranged along a closed circuit for the execution of a refrigeration cycle, said four service components being divided between a first functional unit (16), comprising at least said evaporator (14), and a second functional unit (17), distinct from said first functional unit (16) and comprising at least said compressor (11) and said condenser (12), said first functional unit (16) being accommodatable in the living unit (101) of the vehicle (100), said second functional unit (17) being accommodatable in an engine compartment (102) of the vehicle (100),
**characterized in that** it comprises a sterilizer (18) configured for the sterilization of the air treated by said evaporator (14).

2. The service air conditioning system according to claim 1, **characterized in that** said sterilizer (18) comprises a catalytic filter (19) and an LED light source (20), configured for the activation of a reaction on said catalytic filter (19), which is adapted to sterilize the air.

3. The service air conditioning system according to claim 2, **characterized in that** said LED light source (20) is of the ultraviolet radiation type.

4. The service air conditioning system according to one or more of the preceding claims, **characterized in that** said sterilizer (18) is mounted at the point of ingress of the air, in a fan (14a) of said evaporator (14).

5. The service air conditioning system according to claim 4, **characterized in that** said LED light source (20) is actuated simultaneously with said fan (14a).

6. The service air conditioning system according to one or more of the preceding claims, **characterized in that** it comprises a current inverter which is functionally associated with said compressor (11) and can be connected to an alternator of the vehicle (100), for powering said compressor (11) when the vehicle (100) is underway.

7. A vehicle, of the type provided with a living unit (101) and with an engine compartment (102), which comprises a primary air conditioning system (20), operable only with the engine running and comprising at least four primary components constituted by a compressor (21), a condenser (22), a throttle element (23), and an evaporator (24), **characterized in that** it comprises a service air conditioning system (10) according to one or more of the preceding claims, operable with the engine switched off.

8. The vehicle according to claim 7, **characterized in that** said four service components are distinct from the respective said four primary components.

9. The vehicle according to claim 7, **characterized in that** said compressor (11) of said service system (10) is arranged in parallel with the compressor (21) of said primary system (20), in order to use said condenser (22), said throttle element (23), and said evaporator (24) of said primary system (20) also, respectively, as said condenser (12), said throttle element (13), and said evaporator (14) of said service system (10).

10. The vehicle according to claim 7, **characterized in that** said compressor (11) and said condenser (12) of said service system (10) are arranged in parallel with said compressor (21) and with said condenser (22) of said primary system (20), in order to use said throttle element (23) and said evaporator (24) of said primary system (20) also, respectively, as said throttle element (13) and said evaporator (14) of said service system (10).

11. The vehicle according to claim 7, **characterized in that** said compressor (11) and said evaporator (14) of said service system (10) are arranged in parallel with said compressor (21) and with said evaporator (24) of said primary system (20), in order to use said condenser (22) and said throttle element (23) of said primary system (20) also, respectively, as said condenser (12) and said throttle element (13) of said service system (10).

12. A method for installing a service air conditioning system (10), operable with the engine switched off, in a vehicle (100) provided with a living unit (101) and with a primary air conditioning system (20), operable only with the engine running, said service air conditioning system and said primary air conditioning system (10, 20) each comprising at least four components, constituted by a compressor (11, 21), a condenser (12, 22), a throttle element (13, 23), and an evaporator (14, 24), arranged along respective closed circuits for the execution of a refrigeration cycle, the method entailing arranging a first functional unit (16) of the service system (10), comprising at least the evaporator (14), in the living unit (101) of the vehicle (100), and a second functional unit (17) of the service system (10), distinct from the first functional unit (16) and comprising at least the compressor (11) and the condenser (12), in an engine compartment (102) of the vehicle (100),
**characterized in that** it entails equipping the service system (10) with a sterilizer (18) configured for the sterilization of the air treated by the respective evaporator (14).
